# EUROPEAN PATENT SPECIFICATION

(11) **EP 4 221 598 B1**
(45) Date of publication and mention of the grant of the patent: **09.12.2026**
(21) Application number: 21782968.8
(22) Date of filing: 24.09.2021
(51) Int. Cl.: A61B 8/08, A61B 8/00

(54) **SYSTEMS AND METHODS OF PROVIDING VISUALIZATION AND QUANTITATIVE IMAGING**
SYSTEME UND VERFAHREN ZUR BEREITSTELLUNG VON VISUALISIERUNG UND QUANTITATIVER BILDGEBUNG
SYSTÈMES ET PROCÉDÉS PERMETTANT UNE VISUALISATION ET IMAGERIE QUANTITATIVE

(30) Priority: 30.09.2020 US 202063085315 P
(43) Date of publication of application: 09.08.2023
(73) Proprietor: Koninklijke Philips N.V., 5656 AE Eindhoven (NL)
(72) Inventor: SHI, William Tao, 5656 AE Eindhoven (NL); XIE, Hua, 5656 AE Eindhoven (NL); CARRASCAL, Carolina Amador, 5656 AE Eindhoven (NL); NGUYEN, Man, 5656 AE Eindhoven (NL); YANG, Zhaowen, 5656 AE Eindhoven (NL); TREMBLAY-DARVEAU, Charles, 5656 AE Eindhoven (NL); OLSSON, Lars Jonas, 5656 AE Eindhoven (NL); CHEREPAKHIN, Alexey Viktorovich, 5656 AE Eindhoven (NL); KINCY, Bruce Adrian, 5656 AE Eindhoven (NL); SHAMDASANI, Vijay Thakur, 5656 AE Eindhoven (NL)
(74) Representative: Philips Intellectual Property & Standards
(86) International application number: PCT/EP2021/076288
(87) International publication number: WO 2022/069352

(56) References cited:
- US-A1- 2012 116 218
- US-A1- 2017 273 667
- US-A1- 2019 200 962
- US-B2- 8 167 803

## Description

### TECHNICAL FIELD

This application relates to providing imaging data for visualization purposes and imaging data for quantitative purposes. More specifically, this application relates to providing ultrasound data for generating an image for display and providing ultrasound data for calculating a parameter, such as a physiologically relevant index.

### BACKGROUND

US 2019/200962 A1 discloses an ultrasound imaging apparatus and control method thereof to improve the diagnosis of fatty liver disease.

US 2017/273667 A1 discloses methods for backscatter imaging.

Non-alcoholic Fatty Liver Disease (NAFLD) has become one of the major causes of liver disease due to high prevalence of obesity and diabetes. Its incidence rate has been steadily increasing affecting about 25%-30% population in western and developing countries. The clinical term for fatty liver is hepatic steatosis, defined as excessive accumulation of fat (above 5%-10% by weight) in liver cells as triglycerides. Early stage of liver steatosis is silent and reversible by simple lifestyle change, for instance through regular exercise and healthy dieting. Liver steatosis can turn into more advanced liver disease such as non-alcoholic steatohepatitis (NASH) and liver fibrosis. If left untreated at these stages, fatty liver will progress to end-stage disease including cirrhosis and primary cancer hepatocellular carcinoma.

In current clinical practice, the gold standard of fatty liver diagnosis is liver biopsy, an invasive procedure subject to sampling error and interpretation variability. Magnetic resonance proton density fat fraction (MR-PDFF) is considered the new reference standard for NAFLD diagnosis as it can provide a quantitative biomarker of liver fat content. However, MR-PDFF is an expensive diagnostic tool which may not be available at every hospital. Compared to MR, ultrasound is a widely available and cost-effective imaging modality, more suitable for screening general population and low-risk groups.

Hepato-renal index (HRI), an ultrasound-based method, has been used clinically for fatty liver detection. Excessive fat infiltration in liver increases acoustic backscattering coefficient leading to higher grayscale values in ultrasound B-mode imaging. At a normal state, liver parenchyma and renal cortex have similar echogenicity. With more fat deposit, liver will appear more hyperechoic (i.e. brighter) than kidney cortex. HRI is often calculated as the echo-intensity ratio of liver to kidney. Based on the B-mode data echo intensities from the liver and kidney are estimated by selecting regions of interest (ROIs) within the liver parenchyma and the kidney cortex at a similar depth and then averaging grayscale echo-intensity values in the ROIs. However, there are reliability issues with HRI that limit its application-mainly due to the non-linear relationship between the display intensity (logarithmically scaled or grayscale) and the true in-situ echo intensity. Also, non-ideal selection of the ROIs can also impact reliability and reproducibility of the HRI. Accordingly, improved techniques for acquiring the HRI are desired.

### SUMMARY

The invention is defined by the independent claims. Dependent claims define advantageous embodiments.

Systems and methods for providing medical imaging data for visualization and quantification are disclosed. In some examples, medical imaging data for visualization may be acquired and/or processed to provide an image to a user that has a high image quality (e.g., low signal-to-noise, high contrast, reduced or no artifacts). The user may select regions of interest (ROIs) from the image via a user interface. Medical imaging data for quantification may be acquired and/or processed to provide data that provides more accurate, reliable, and/or quantifiable results for one or more calculations. In some examples, the medical imaging data may be acquired and/or processed in a manner that does not introduce unknown relationships between signal intensities and resulting medical imaging data (e.g., nonlinearities). In some examples, the medical imaging data for visualization may be registered to the medical imaging data for quantification. Thus, when a user selects an ROI in the image generated from the medical imaging data for visualization, medical imaging data for quantification that corresponds to the ROI is used for calculating a parameter such as a physiologically index, for example, a hepato-renal index.

### BRIEF DESCRIPTION OF THE DRAWINGS

**FIG. 1** is a block diagram of an ultrasound imaging system arranged according to principles of the present disclosure.
**FIG. 2** is a block diagram illustrating an example processor according to principles of the present disclosure.
**FIG. 3** is a graphical depiction of a data processing path according to an example of the present disclosure.
**FIG. 4** is a graphical depiction of a data processing path according to an example of the present disclosure.
**FIG. 5** illustrates example images generated from visualization data and quantification data according to principles of the present disclosure.
**FIG. 6** illustrates example images generated from visualization data and quantification data on a display according to principles of the present disclosure.
**FIG. 7** is a flow chart of a method in accordance with principles of the present disclosure.

### DESCRIPTION

The following description of certain exemplary examples is merely exemplary in nature and is in no way intended to limit the disclosure or its applications or uses. In the following detailed description of examples of the present systems and methods, reference is made to the accompanying drawings which form a part hereof, and in which are shown by way of illustration specific examples in which the described systems and methods may be practiced. These examples are described in sufficient detail to enable those skilled in the art to practice the presently disclosed systems and methods, and it is to be understood that other examples may be utilized, and that structural and logical changes may be made without departing from the scope of the present disclosure. Moreover, for the purpose of clarity, detailed descriptions of certain features will not be discussed when they would be apparent to those with skill in the art so as not to obscure the description of the present disclosure. The following detailed description is therefore not to be taken in a limiting sense, and the scope of the present systems and methods is defined only by the appended claims.

The hepato-renal index (HRI) is typically acquired based on the pixel intensities of the B-mode image displayed on an ultrasound imaging system. HRI can be a powerful diagnostic indicator for fatty liver disease when it is measured properly using a well-defined grayscale imaging mode with optimal instrumental settings of gain, time gain control (TGC), dynamic range, display map (ideally, linear conversion of echo intensities from decibel scale to grayscale), etc. The reliability of HRI may suffer from a severely nonlinear relationship between a logarithmically scaled (or often even more complexed) and further threshold-limited grayscale display (0-255) versus the true in-situ echo intensity of B-mode image data. Accordingly, specifically designed imaging modes and/or processing methods may be desirable for calculating HRI. For example, fundamental imaging, analysis of the radiofrequency (RF) data, and/or adequately linearized imaging data may be used to calculate HRI.

Another issue impacting the reliability of HRI is improper selection of regions of interest (ROIs) on the liver and/or kidney. ROIs are preferably located on the liver and kidney at locations of high homogeneity (e.g., little to no brighter or dimmer pixels) and unimpeded acoustic paths (e.g., no beam distortion or shadowing). Accordingly, imaging modes and/or processing methods that provide high quality images (e.g., low artifacts, high signal to noise) may be desirable to provide enhanced visualization for a user to facilitate proper selection of the ROIs. For example, harmonic imaging and/or non-linear processing may be performed to provide the high-quality images.

However, the imaging modes and/or processing that provide improved visualization for the user to select the ROIs may introduce nonlinearities or unknown relationships between the ultrasound signals, which may make calculation of the HRI impossible or unreliable. On the other hand, the imaging modes and/or processing that may provide improved quantification of the HRI may produce images that are difficult for a user to interpret, which may lead to non-ideal selection of the ROIs.

The present disclosure is directed to systems and methods for generating separate sets of medical imaging data: one set for visualization and one set for quantification. The medical imaging data for visualization may be generated by imaging modes and/or processing for generating high quality images that facilitate a user's ability to interpret the images. The medical imaging data for quantification may be generated by imaging modes and/or processing for generating medical imaging data that has known relationships (e.g., linear) to signals received from a subject (e.g., echoes responsive to ultrasound signals). In some examples, the same imaging modes may be employed for generation of one set of medical imaging data for visualization and another set of medical imaging data for quantification, but the medical imaging data undergoes different processing processes. Different imaging modes are utilized by interleaving sequences, for generation of two sets of different medical imaging data, respectively, for visualization and for quantification using the same or different processing processes. The medical imaging data for visualization is used to provide an image to a user, for example, to select ROIs, and medical imaging data for quantification corresponding to locations indicated by the ROIs is analyzed to determine a parameter, such as HRI. Thus, in some applications, selection of ROIs may be improved while reliability of the HRI may be maintained or improved.

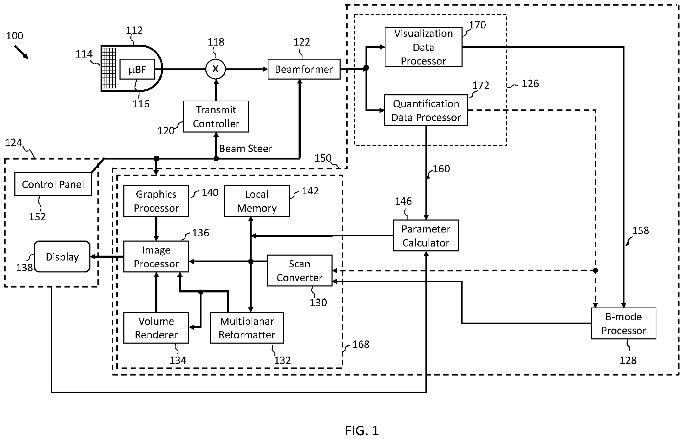

FIG. 1 shows a block diagram of an ultrasound imaging system 100 constructed in accordance with the principles of the present disclosure. An ultrasound imaging system 100 according to the present disclosure may include a transducer array 114, which may be included in an ultrasound probe 112. In other examples, the transducer array 114 may be in the form of a flexible array configured to be conformally applied to a surface of subject to be imaged (e.g., patient). The transducer array 114 is configured to transmit ultrasound signals (e.g., waveforms) and receive echoes (e.g., reflected and/or backscattered ultrasound signals) responsive to the transmitted ultrasound signals at various directions (e.g., beams). A variety of transducer arrays may be used, e.g., linear arrays, curved arrays, or phased arrays. The transducer array 114, for example, can include a two-dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some examples, the transducer array 114 may be coupled to a micro-beamformer 116, which may be located in the ultrasound probe 112, and which may control the transmission and reception of signals by the transducer elements in the array 114. In some examples, the micro-beamformer 116 may control the transmission and reception of signals by active elements in the array 114 (e.g., an active subset of elements of the array that define the active aperture at any given time).

In some examples, the micro-beamformer 116 may be coupled, e.g., by a probe cable or wirelessly, to a transmit/receive (T/R) switch 118, which switches between transmission and reception and protects the main beamformer 122 from high energy transmit signals. In some examples, for example in portable ultrasound systems, the T/R switch 118 and other elements in the system can be included in the ultrasound probe 112 rather than in the ultrasound system base, which may house the image processing electronics. An ultrasound system base typically includes software and hardware components, which may include circuitry, for signal processing and image data generation as well as executable instructions for providing a user interface.

The transmission of ultrasonic signals from the transducer array 114 under control of the micro-beamformer 116 is directed by the transmit controller 120, which may be coupled to the T/R switch 118 and a main beamformer 122. The transmit controller 120 may control characteristics of the ultrasound signal waveforms transmitted by the transducer array 114, for example, amplitude, phase, and/or polarity. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. For example, the user may select whether the transmit controller 120 causes the transducer array 114 to operate in a harmonic imaging mode, fundamental imaging mode, Doppler imaging mode, or a combination of imaging modes (e.g., interleaving different imaging modes). The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some examples, the partially beamformed signals produced by the micro-beamformer 116 may be coupled to a main beamformer 122 where partially beamformed signals from individual patches of transducer elements may be combined into a fully beamformed signal. In some examples, micro-beamformer 116 is omitted, and the transducer array 114 is under the control of the beamformer 122 and beamformer 122 performs all beamforming of signals. In examples with and without the micro-beamformer 116, the beamformed signals of beamformer 122 are coupled to processing circuitry 150, which may include one or more processors (e.g., a signal processor 126, a B-mode processor 128, RF image processor 144, parameter calculator 146, and one or more image generation and processing components 168) configured to produce an ultrasound image from the beamformed signals (e.g., beamformed RF data).

In signal processor 126 may receive the beamformed RF data and process the received beamformed RF data in various ways, such as bandpass filtering, decimation, and I and Q component separation. The signal processor 126 may also perform additional signal enhancement such as speckle reduction, signal compounding, and electronic noise elimination. The processing of the beamformed RF data performed by the signal processor 126 may be different based, at least in part, on whether visualization data (e.g., medical imaging data for visualization) and/or quantification data (e.g., medical imaging data for quantification) is desired. As shown in FIG. 1, the signal processor 126 includes visualization data processor 170 to generate visualization data and quantification data processor 172 to generate quantification data by processing the beamformed RF data.

The visualization data processor 170 may process the beamformed RF data to generate visualization data that may be used to generate an image with enhanced quality compared to raw image data (e.g., improved signal to noise ratio, reduces artifacts, improves contrast). In some embodiments, the visualization data processor 170 may perform processing operations (e.g., nonlinear operations) which, in some cases, may cause the visualization data to lose a linear and/or known relationship with the echoes that are directly received by the transducer array 114. Examples of processing operations that may change the relationship with the echoes include, but are not limited to, adjustment of the dynamic range of pixel values, smoothing algorithms, and/or reverberation elimination algorithms. In some examples, homogeneous portions may be clearly visualized and segmented from defects (e.g., vessels and small cavities) in hepatic parenchyma and renal cortex using imaging processing techniques such as speckle smoothing/reduction and/or edge enhancement.

The quantification data processor 172 may process the beamformed RF data to generate quantification data for calculating parameters, including physiologically relevant parameters such as HRI. The quantification data processor 172 may process the beamformed RF data such that the relationships between the echo signals received by the transducer array 114 and the quantification data are linearly proportional and/or known. In some embodiments, the relationship between the echo signals and the quantification data are linear. Examples of processing operations that may maintain the relationship include, but are not limited to, fixed gain and fixed time gain compensation.

The beamformed RF data provided to the visualization data processor 170 is different from the beamformed RF data provided to the quantification data processor 172. Beamformed RF data acquired by one imaging mode is provided to the visualization data processor 170 and data acquired by another imaging mode is provided to the quantification data processor 172. Different imaging modes may cause the transducer array 114 to transmit ultrasound signals and receive the echoes using different settings (e.g., bandwidth, center frequency, power, number of transmit pulses, number and locations of transmit and receive transducer elements). Frames (e.g., image slices) for two different imaging modes are alternated (e.g., interleaved) during acquisition. Interleaving the imaging modes helps to ensure that the frames acquired in the different imaging mode spatially correspond to one another. That is, movement of the probe and/or subject is minimal between frames of differing imaging modes. In some embodiments, the imaging modes and the pattern with which the imaging modes are applied by the transducer array 114 may be based on control signals provided by the transmit controller 120.

In some embodiments, the imaging mode used to provide beamformed RF data for the visualization data processor 170 may be an imaging mode that may provide visualization data that can be used to generate high quality images, for example, harmonic imaging. In some embodiments, the imaging mode used to provide beamformed RF data for the quantification data processor 172 may be an imaging mode that may preserve the relationship between the echo signals and the quantification data, for example, fundamental imaging. While fundamental and harmonic imaging modes are provided as examples, the principles of the present disclosure are not limited to these imaging modes. When different beamformed RF data is provided to the visualization processor 170 and the quantification processor 172, the processing performed on the beamformed RF data by the visualization processor 170 and the quantification processor 172 may be the same or different.

Although visualization processor 170 and quantification processor 172 are shown as separate components, in some embodiments, the visualization processor 170 and the quantification processor 172 may be implemented by a single processor, particularly when beamformed RF data is provided sequentially from interleaved imaging modes. However, a single processor capable of parallel processing may be used when the same beamformed RF data is provided to the visualization processor 170 and the quantification processor 172 in some embodiments. Alternatively, each of the visualization processor 170 and the quantification processor 172 may be implemented by multiple processors (e.g., multiple graphical processing units) in some embodiments.

Some of the processed signals output from the signal processor 126 (e.g., I and Q components or IQ signals of the visualization data generated by the visualization data processor 170) may be coupled to additional downstream signal processing components (e.g., hardware and/or software) for image generation. The IQ signals may be coupled to one or more signal paths within the system, each of which may be associated with a specific arrangement of signal processing components suitable for generating different types of image data (e.g., amplitude-based B-mode image data, phase-based Doppler image data). For example, the system may include a B-mode signal path 158 which couples the signals from the signal processor 126 to a B-mode processor 128 for producing B-mode image data. The B-mode processor 128 can employ amplitude detection for the imaging of structures in the body.

Some of the processed signals output from the signal processor 126 (e.g., quantification data generated by the quantification data processor 172) may be coupled to additional downstream data processing circuits for analysis. For example, the system may include a parameter calculation path 160 which couples the signals from the signal processor 126 to a parameter calculator 146 for calculating one or more parameters, such as HRI.

Optionally, the quantification data may also be provided to the B-mode processor 128 and/or scan converter 130. As will be described herein, in some embodiments, an image based on the quantification data may be provided to a user in addition to the image based on the visualization data.

The signals produced by the B-mode processor 128 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two-dimensional (2D) sector-shaped format, or a pyramidal or otherwise shaped three-dimensional (3D) format.

The multiplanar reformatter 132 can convert echoes which are received from points in a common plane in a volumetric region of the body into an ultrasonic image (e.g., a B-mode image, harmonic image, fundamental image, blended image) of that plane, for example as described in U.S. Pat. No. 6,443,896 (Detmer). The scan converter 130 and multiplanar reformatter 132 may be implemented as one or more processors in some examples.

A volume renderer 134 may generate an image (also referred to as a projection, render, or rendering) of the 3D dataset as viewed from a given reference point, e.g., as described in U.S. Pat. No. 6,530,885 (Entrekin et al.). The volume renderer 134 may be implemented as one or more processors in some examples. The volume renderer 134 may generate a render, such as a positive render or a negative render, by any known or future known technique such as surface rendering and maximum intensity rendering.

Output (e.g., B-mode images) from the scan converter 130, the multiplanar reformatter 132, and/or the volume renderer 134 may be coupled to an image processor 136 for further enhancement, buffering and temporary storage before being displayed on an image display 138. For example, an image generated from the visualization data generated by the visualization processor 170 may be provided to the user on the display.

A graphics processor 140 may generate graphic overlays for display with the images. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 124 can also be coupled to the multiplanar reformatter 132 for selection and control of a display of multiple multiplanar reformatted (MPR) images.

The system 100 may include local memory 142. Local memory 142 may be implemented as any suitable non-transitory computer readable medium (e.g., flash drive, disk drive). Local memory 142 may store data generated by the system 100 including images, calculated parameters, executable instructions, inputs provided by a user via the user interface 124, or any other information necessary for the operation of the system 100.

As mentioned previously system 100 includes user interface 124. User interface 124 may include display 138 and control panel 152. The display 138 may include a display device implemented using a variety of known display technologies, such as LCD, LED, OLED, or plasma display technology. In some examples, display 138 may comprise multiple displays. The control panel 152 may be configured to receive user inputs (e.g., selection of imaging modes, selection of regions of interest). The control panel 152 may include one or more hard controls (e.g., buttons, knobs, dials, encoders, mouse, trackball or others). In some examples, the control panel 152 may additionally or alternatively include soft controls (e.g., GUI control elements or simply, GUI controls) provided on a touch sensitive display. In some examples, display 138 may be a touch sensitive display that includes one or more soft controls of the control panel 152.

According to principles of the present disclosure, the system 100 may include a parameter calculator 146. The parameter calculator 146 may be implemented in software, hardware, or a combination thereof. For example, the parameter calculator 146 may be implemented by a processor that executes instructions for calculating one or more parameters, including HRI. The parameter calculator 146 may receive the quantification data from the quantification data processor 172. The parameter calculator 146 may receive indications of selections of ROIs from the user interface 124 based on the image generated from the visualization data. Where the parameter to be calculated is HRI, one of the ROIs may be from an area of an image corresponding to the hepatic parenchyma and another ROI may be from an area of the image corresponding to the renal cortex. The parameter calculator 146 may use the quantification data acquired from the regions corresponding to the ROIs to calculate one or more HRIs. The parameter calculated by the parameter calculator 146 may be provided to the local memory 142 for storage and/or to the image processor 136 for displaying to the user on display 138.

The user interface 124 may be used to adjust various parameters of image acquisition, generation, and/or display. For example, a user may select an imaging mode, adjust the power, adjust a level of gain, dynamic range, turn on and off spatial compounding, and/or level of smoothing. In some embodiments, the user-adjustable settings may affect the imaging mode and/or processing of the visualization data (e.g., beamformed RF data processed by the visualization processor 170). In some embodiments, the user-adjustable settings may not affect the imaging mode and/or processing of the quantification data (e.g., beamformed RF data processed by the quantification processor 172). This may allow the system 100 to provide an image for display this is adjustable by the user without changing the quantification data. This may help ensure that the linear and/or known relationships of the quantification data with the echo signals is preserved regardless of user adjustments to the displayed image. In some applications, this may prevent parameters calculated by the parameter calculator 146 from the quantification data from being compromised (e.g., less accurate or unreliable).

In some examples, various components shown in FIG. 1 may be combined. For instance, image processor 136 and graphics processor 140 may be implemented as a single processor. In another example, the parameter calculator 146 and the signal processor 126 may be implemented as a single processor. In some examples, various components shown in FIG. 1 may be implemented as separate components. For example, signal processor 126 may be implemented as separate signal processors for each imaging mode (e.g., fundamental or harmonic grayscale mode). In some examples, one or more of the various processors shown in FIG. 1 may be implemented by general purpose processors and/or microprocessors configured to perform the specified tasks (e.g., grayscale modes designed for a greater penetration or higher resolution). In some examples, one or more of the various processors may be implemented as application specific circuits (ASICS) or other components. In some examples, one or more of the various processors (e.g., image processor 136) may be implemented with one or more graphical processing units (GPU).

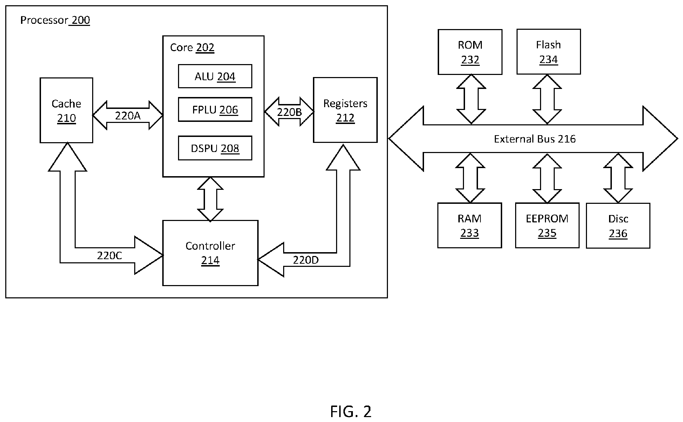

FIG. 2 is a block diagram illustrating an example processor 200 according to principles of the present disclosure. Processor 200 may be used to implement one or more processors described herein, for example, image processor 136 shown in FIG. 1. Processor 200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 200 may include one or more cores 202. The core 202 may include one or more arithmetic logic units (ALU) 804. In some examples, the core 202 may include a floating point logic unit (FPLU) 206 and/or a digital signal processing unit (DSPU) 208 in addition to or instead of the ALU 204.

The processor 200 may include one or more registers 212 communicatively coupled to the core 202. The registers 212 may be implemented using dedicated logic gate circuits (e.g., flip-flops) and/or any memory technology. In some examples the registers 212 may be implemented using static memory. The register may provide data, instructions and addresses to the core 202.

In some examples, processor 200 may include one or more levels of cache memory 210 communicatively coupled to the core 202. The cache memory 210 may provide computer-readable instructions to the core 202 for execution. The cache memory 210 may provide data for processing by the core 202. In some examples, the computer-readable instructions may have been provided to the cache memory 210 by a local memory, for example, local memory attached to the external bus 3216. The cache memory 210 may be implemented with any suitable cache memory type, for example, metal-oxide semiconductor (MOS) memory such as static random access memory (SRAM), dynamic random access memory (DRAM), and/or any other suitable memory technology.

The processor 200 may include a controller 314, which may control input to the processor 200 from other processors and/or components included in a system (e.g., control panel 152 and scan converter 130 shown in FIG. 1) and/or outputs from the processor 200 to other processors and/or components included in the system (e.g., display 138 and volume renderer 134 shown in FIG. 1). Controller 214 may control the data paths in the ALU 204, FPLU 206 and/or DSPU 208. Controller 214 may be implemented as one or more state machines, data paths and/or dedicated control logic. The gates of controller 214 may be implemented as standalone gates, FPGA, ASIC or any other suitable technology.

The registers 212 and the cache memory 210 may communicate with controller 214 and core 202 via internal connections 220A, 220B, 220C and 220D. Internal connections may be implemented as a bus, multiplexor, crossbar switch, and/or any other suitable connection technology.

Inputs and outputs for the processor 200 may be provided via a bus 216, which may include one or more conductive lines. The bus 216 may be communicatively coupled to one or more components of processor 200, for example the controller 214, cache 210, and/or register 212. The bus 216 may be coupled to one or more components of the system, such as display 138 and control panel 152 mentioned previously.

The bus 216 may be coupled to one or more external memories. The external memories may include Read Only Memory (ROM) 232. ROM 232 may be a masked ROM, Electronically Programmable Read Only Memory (EPROM) or any other suitable technology. The external memory may include Random Access Memory (RAM) 233. RAM 233 may be a static RAM, battery backed up static RAM, Dynamic RAM (DRAM) or any other suitable technology. The external memory may include Electrically Erasable Programmable Read Only Memory (EEPROM) 235. The external memory may include Flash memory 234. The external memory may include a magnetic storage device such as disc 236. In some examples, the external memories may be included in a system, such as ultrasound imaging system 100 shown in Fig. 1, for example local memory 142.

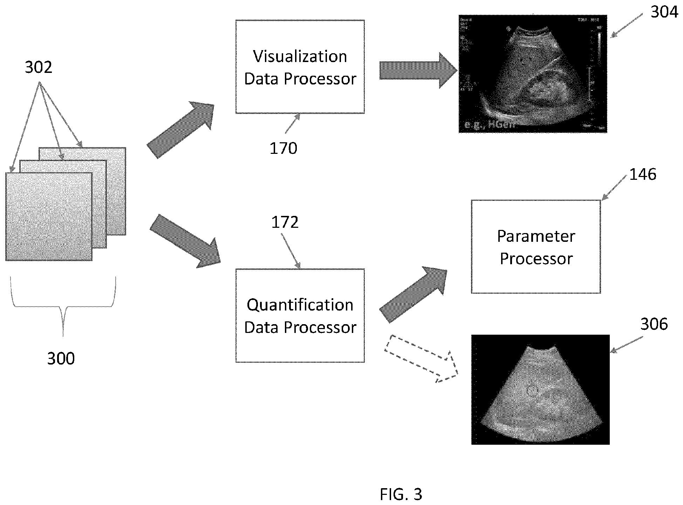

**FIG. 3** is a graphical depiction of a data processing path according to an example of the present disclosure. Multiple ultrasound transmit/receive events may be performed, for example, by transducer array 114, to acquire imaging data 300. The imaging data 300 is illustrated in FIG. 3 as multiple image frames 302. However, this is only for easy of illustration, and imaging data 300 may be RF data not yet processed and/or arranged into image frames at this point in the data path. In some examples, each of the image frames 302 may have been acquired by a separate transmit/receive event. The imaging data 300 may be beamformed, for example, by microbeam-former 116 and/or main beamformer 122. After beamforming, the image data 300 may be provided to visualization data processor 170 and quantification data processor 172. The visualization data processor 170 may process the imaging data 300 to generate visualization data that may be used to generate an image 304. In some examples, the image 302 may be provided on display 138. The quantification data processor 170 may process the imaging data 300 to generate quantification data that may be provided to a parameter processor 146. The parameter processor 146 may calculate one or more parameters based on the quantification data, including the hepato-renal index. Optionally, in some examples, the quantification data may also be used to generate an image 306 and displayed on display 138. Generating the image 306 from the quantification data may include further processing and/or arranging of the quantification data by the B-mode processor 128 and/or scan converter 130 in some examples.

As discussed with reference to FIG. 1, when the same imaging data 300 is provided to both the visualization data processor 170 and the quantification data processor 172, the imaging data 300 may be processed differently by the two processors. For example, the visualization data processor 170 may perform non-linear operations on the imaging data 300 while quantification data processor 172 may perform linear operations.

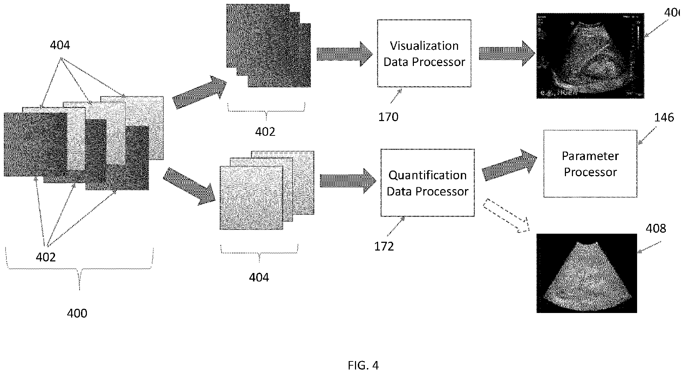

**FIG. 4** is a graphical depiction of a data processing path according to an example of the present disclosure. Multiple ultrasound transmit/receive events may be performed, for example, by transducer array 114, to acquire imaging data 400. The imaging data 400 is illustrated in FIG. 4 as multiple image frames 402 and image frames 404. However, this is only for easy of illustration, and imaging data 400 may be RF data not yet processed and/or arranged into image frames at this point in the data path. In some examples, each of the image frames 402 and each of the image frames 404 may have been acquired by a separate transmit/receive event. The image frames 402 and image frames 404 are acquired by different imaging modes. For example, a harmonic-dominant imaging mode with adequately high resolution (e.g., high line density and high frequency) may be used and processed with a combination of various image enhancement technologies such as speckle reduction and edge/vessel enhancement; while a moderate resolution (e.g., relatively low line density and frequency) fundamental mode, with low beam distortion and reverberation in an intermediate imaging depth, may be utilized for the generation of the image frames 404. Although the imaging modes are shown as interleaved such that the imaging mode changes with each consecutive image frame, other acquisition patterns may be used in other examples. The imaging data 400 may be beamformed, for example, by micro-beamformer 116 and/or main beamformer 122. Visualization processor 170 and quantification processor 172 receive different portions of the imaging data 400. Before or after beamforming, the image frames 402 may be separated from image frames 404. Image frames 402 are provided to visualization processor 170 and image frames 404 are provided to quantification processor 172. Thus, the visualization processor 170 receives a portion of imaging data 400 acquired by one imaging mode while quantification processor 172 receives a portion of imaging data 400 acquired by another imaging mode.

The visualization data processor 170 may process the imaging data 400 corresponding to image frames 402 to generate visualization data that may be used to generate an image 406. In some examples, the image 406 may be provided on display 138. The quantification data processor 170 may process the imaging data 400 corresponding to image frames 404 to generate quantification data that may be provided to a parameter processor 146. The parameter processor 146 may calculate one or more parameters based on the quantification data, including the hepato-renal index. Optionally, in some examples, the quantification data may also be used to generate an image 408 and displayed on display 138. Generating the image 408 from the quantification data may include further processing and/or arranging of the quantification data by the B-mode processor 128 and/or scan converter 130 in some examples.

As discussed with reference to FIG. 1, in some examples, when different imaging data (e.g., images acquired by different imaging modes) is provided to the visualization data processor 170 and the quantification data processor 172, the imaging data 400 may be processed the same by both processors, and the properties of the visualization data and the quantification data may be based on the imaging mode. However, in other examples, the different imaging data may be processed differently by the two processors. For example, the visualization data processor 170 may perform non-linear operations on the image frames 402 of imaging data 400 while quantification data processor 172 may perform linear operations on image frames 404 of imaging data 400. In these examples, the properties of the visualization data and quantification data may be based on the imaging mode and the processing techniques.

The example illustrated and described with reference to FIG. 3 may provide a higher frame rate and/or may be relatively easier to implement than the example illustrated and described with reference to FIG. 4. However, the example shown in FIG. 4 may provide better optimized imaging settings (e.g., frequency settings, line density) for generating each of the visualization data and the quantification data.

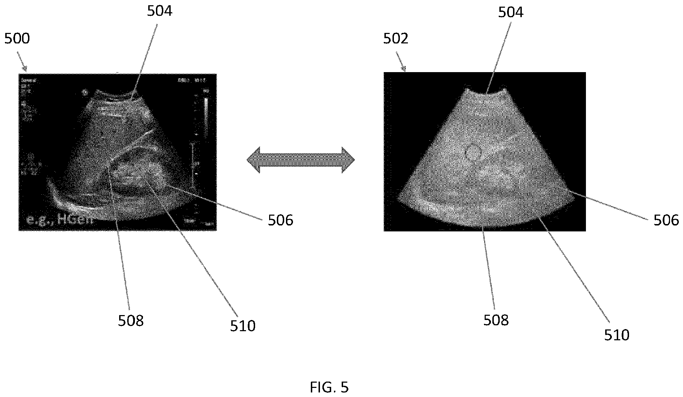

**FIG. 5** illustrates example images generated from visualization data and quantification data according to principles of the present disclosure. The image 500 is an ultrasound image generated from beamformed RF data processed by non-linear processing methods (e.g., altering dynamic range) and/or by a non-linear imaging mode (e.g., harmonic imaging). The image 502 is an ultrasound image generated from beamformed RF data processed by linear processing methods and/or by a linear imaging mode (e.g., fundamental imaging). Both image 500 and 502 are generated from ultrasound data of an image slice acquired from a same spatial location at a liver 504 and a kidney 506. In some examples, image 500 may have been generated from data provided by visualization data processor 170 and image 502 may have been generated from data provided by quantification data processor 172.

The contrast of image 500 is greater than the contrast of image 502, and the speckle noise in image 500 is less than the speckle noise in image 502. Accordingly, it may be easier for a user to discern what areas of the liver 504 and kidney 506 are suitably homogenous for calculating a physiological parameter, such as HRI. A first region of interest (ROI) 508 on the liver 504 and a second ROI 510 on the kidney 506 may be selected by a user, for example, by providing user inputs via user interface 124. In some examples, the ROIs 508, 510 may be selected by the user by selecting locations on image 500 while viewing image 500 on a display, such as display 138. In some examples, image 502 may not be provided to the user on the display. In other examples, the ROIs 508, 510 may be selected by the imaging system and/or at least with partial assistance by the system.

In this example, although the ROIs 508, 510 are selected based on image 500, the HRI may be calculated based on the corresponding data of the ROIs 508, 510 of image 502. In some applications, the HRI value calculated from the quantification data of image 502 may be more reliable than an HRI value calculated from the visualization data of image 500.

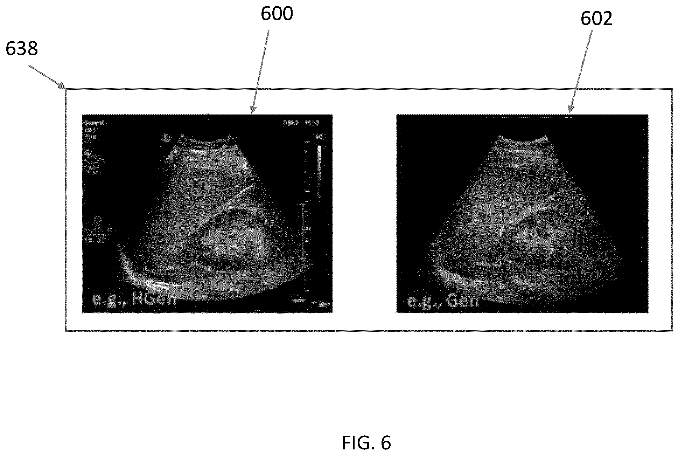

Optionally, an image generated from quantification data may also be provided on a display. **FIG. 6** illustrates example images generated from visualization data and quantification data on a display according to principles of the present disclosure. Display 638 may be used to implement display 138 in some examples. Display 638 may provide images 600 and 602. The image 600 is an ultrasound image generated from beamformed RF data processed by non-linear processing methods and/or by a non-linear imaging mode. The image 602 is an ultrasound image generated from beamformed RF data processed by linear processing methods and/or by a linear imaging mode. The image 600 is an image generated from visualization data that may be generated using different signal processing on the acquisition data used to generate image 602. The images 600 and 602 may be shown or displayed concurrently to the user, e.g., as shown in FIG. 6. In other examples, a toggle control may be provided on the user interface (e.g., user interface 124) to enable the user to toggle between the two images by operating the toggle control on the user interface. Although shown the same size in FIG. 6, in other examples, one of the images, such as image 402 may be smaller on the display than the other image (e.g., image 600). In some examples, only the image generated from visualization data (e.g., image 600 or 500 in the example in FIG. 5) may be displayed to the user, such as to enable the user to select the ROIs and/or visually confirm the location of ROIs selected by the system and/or with assistance by the system.

In some examples, the generating of quantification data for the second image (e.g., image 602) may include additional processing, such as log-compressed dynamic range processing. In other examples, the "native" quantification data may be used to generate image 602, such as image 502. By native data, it is meant that the electrical signals generated by the transducer elements responsive to the echoes have been minimally processed, for example, the signals may be beamformed and a linear gain (e.g., signal amplification) applied. In some examples, if a user adjusts display and/or acquisition settings for image 600, the display and/or acquisition settings for image 602 may not be adjusted (e.g., remain the same).

Providing both image 600 and image 602 may allow a user to confirm the images 600 and 602 are of the same spatial location when the images are generated from different data, such as different acquisitions (e.g., when different imaging modes are interleaved). Providing both image 600 and image 602 may allow the user to confirm no artifacts were introduced by the nonlinear processing and/or otherwise give the user confidence that suitable ROIs have been selected when the images 600 and 602 are generated from different processing of the same data or when generated from different acquisitions.

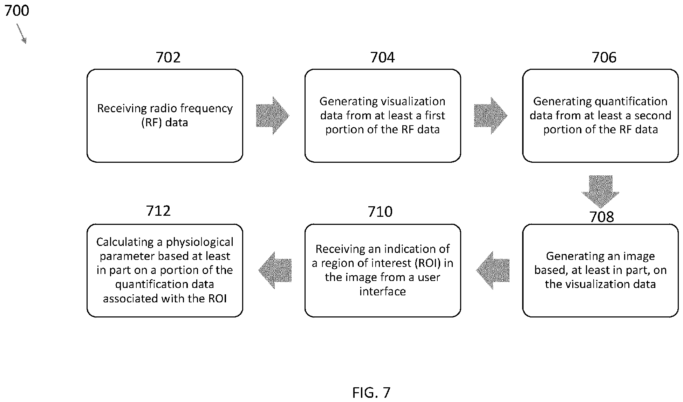

**FIG. 7** is a flow chart of a method in accordance with principles of the present disclosure. The method 700 is performed by an ultrasound imaging system, such as ultrasound imaging system 100.

At block 702, the system (e.g., ultrasound imaging system 100) receives radio frequency (RF) data. The RF data corresponds to echoes generated responsive to ultrasound signals transmitted by an ultrasound transducer array, such as ultrasound transducer array 114. The RF data may be received by a signal processor, such as signal processor 126.

At block 704, visualization data is generated from at least a first portion of the RF data (e.g., image frames 402 in the example shown in FIG. 4). In some examples, the visualization data may be generated by a processor, such as visualization data processor 170. At block 706, quantification data is generated from at least a second portion of the RF data. In some examples, the quantification data may be generated by a processor, such as quantification processor 172. As noted previously, in some examples, the signal processor 126 may be a single processor that generates both the visualization data and the quantification data.

In some examples, generating the visualization data comprises processing the RF data by at least one non-linear processing method, and generating the quantification data comprises processing the RF data by at least one linear processing method. The first portion of the RF data is acquired by the ultrasound transducer array by a first imaging mode, and the second portion of the RF data is acquired by the ultrasound transducer array by a second imaging mode. In some examples, the first imaging mode is a harmonic imaging mode, and the second imaging mode is a fundamental imaging mode. The first and second imaging modes are interleaved.

At block 708, an image is generated from the visualization data. In some embodiments, the image may be generated by one or more processors, such as visualization data processor 170, B-mode processor 128, scan converter 130, multiplanar reformatter 132, volume renderer 134, and/or image processor 136. The image may be provided on a display, such as display 138, in some examples. Optionally, in some examples, a second image is generated from the quantification data. In some examples, one or more processors (e.g., B-mode processor 128) may further process and/or arrange the quantification data to generate the second image (e.g., log-compressed dynamic range matching). In some examples, a user input may be received via the user interface comprising a change to the image and one or more processors may alter the generation of the visualization data responsive to the user input. However, in some examples, the quantification data may remain unchanged.

At block 710, a user provides an indication of a region of interest (ROI) in the image via a user interface. In some examples, the user interface may include user interface 124. At block 712, a processor calculates a physiological parameter based at least in part on a portion of the quantification data associated with the ROI. In some examples, the physiological parameter may be calculated by one or more processors, such as quantification data processor 172 and/or parameter calculator 146. The physiological parameter may be provided on the display and/or saved to a memory, such as local memory 142.

In some examples, a user may provide a second indication of a second ROI via the user interface and the processor may calculate the physiological parameter based at least in part on the portion of the quantification data associated with the ROI and a portion of the quantification data associated with the second ROI. For example, the ROI may correspond to a portion of a liver and the second ROI may correspond to a portion of a kidney and calculating the physiological parameter comprises calculating a ratio of an echo intensity of the liver and an echo intensity of the kidney, that is, the HRI.

Although the example method described with reference to FIG. 7 describes different portions of the imaging data being processed differently, in other examples, such as the one described with reference to FIG. 3, the same imaging data and/or same portion of imaging data may be processed differently to generate the visualization data and the quantification data.

The systems and methods disclosed herein may generate visualization data for generating images to provide on a display and quantification data for calculating one or more parameters. The visualization data and quantification data may be generated by different processing methods and/or imaging modes. In some applications, by providing one set of data for generating an image to display and another set of data for calculating parameters, ROI selection may be improved and/or more reliable parameters may be determined.

In various examples where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure, it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner for combined visualization and quantification. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples or processes described herein may be combined with one or more other examples and/or processes, or be separated and/or performed amongst separate devices or device portions in accordance with the present systems, devices and methods.

Finally, the above discussion is intended to be merely illustrative of the present systems and methods and should not be construed as limiting the appended claims to any particular example or group of examples. Accordingly, the specification and drawings are to be regarded in an illustrative manner and are not intended to limit the scope of the appended claims.

## Claims

1. An ultrasound imaging system (100) comprising:
an ultrasound probe (112) configured to transmit ultrasound signals and receive echoes responsive to the ultrasound signals and provide radio frequency data (400) corresponding to the echoes; and
a processor (126, 170, 172, 200) configured to receive the radio frequency data and further configured to: generate visualization data from a first portion of the radio frequency data, wherein an image (406, 408, 500, 502, 600, 602) is based, at least in part, on the visualization data; generate quantification data from a second portion of the radio frequency data; wherein the quantification data comprises medical imaging data for quantification;
a display (138) configured to display the image; and
a user interface (124) configured to receive indications of a first region of interest (508) in the image, wherein the first region of interest corresponds to a portion of a liver (504); receive indications of a second region of interest (510) in the image, wherein the second region of interest corresponds to a portion of a kidney (506);
and wherein the processor is further configured to: receive the indications of the first and second regions of interest; and calculate a physiological parameter, wherein the physiological parameter includes a hepato-renal index;
and wherein calculating the physiological parameter comprises calculating a ratio of an echo intensity of the liver and an echo intensity of the kidney based at least in part on a portion of the quantification data associated with the first region of interest and a portion of the quantification data associated with the second region of interest;
**characterized in that** the first portion of the radio frequency data is acquired by a first imaging mode and the second portion of the radio frequency data is acquired by a second imaging mode different from the first imaging mode; wherein the first imaging mode is interleaved with the second imaging mode.

2. The ultrasound imaging system (100) of claim 1, wherein the user interface (124) is further configured to receive inputs for adjusting the image (406, 408, 500, 502, 600, 602), and wherein the processor (126, 200) is further configured to adjust the visualization data based on the inputs.

3. The ultrasound imaging system (100) of claim 1, wherein the display (138) is further configured to display a second image generated from the quantification data.

4. The ultrasound imaging system (100) of claim 1, wherein the processor (126, 200) is configured to
generate the visualization data by processing the first portion of the radio frequency data by at least one non-linear processing method, and
generate the quantification data by processing the second portion of the radio frequency data by at least one linear processing method.

5. The ultrasound imaging system (100) of claim 1, wherein the first imaging mode is a harmonic imaging mode, and the second imaging mode is a fundamental imaging mode.

6. An ultrasound imaging method (700) comprising:
receiving (702) radio frequency data (400) corresponding to echoes generated responsive to ultrasound signals transmitted by an ultrasound transducer array (114);
generating (704) visualization data from a first portion (402) of the radio frequency data (400) by a processor;
generating (706) quantification data from a second portion (404) of the radio frequency data (400) by a processor, wherein the quantification data comprises medical imaging data for quantification;
generating (708) an image (406, 500, 600) by a display based, at least in part, on the visualization data;
receiving (710) an indication of a first region of interest (508) in the image from the user interface (124), wherein the first region of interest corresponds to a portion of a liver (504);
receiving (710) an indication of a second region of interest (510) in the image from a user interface (124), wherein the second region of interest corresponds to a portion of a kidney (506); and
calculating a physiological parameter, wherein the physiological parameter comprises a HepatoRenal Index, wherein calculating the physiological parameter comprises calculating ratio of an echo intensity of the liver and an echo intensity of the kidney based at least in part on a portion of the quantification data associated with the first region of interest and a portion of the quantification data associated with the second region of interest, **characterized in that**
the first portion of the radio frequency data is acquired by a first imaging mode, and
the second portion of the radio frequency data is acquired by a second imaging mode different from the first imaging mode, wherein the first imaging mode is interleaved with the second imaging mode.

7. The method (700) of claim 6, wherein generating the visualization data comprises processing the first portion of the radio frequency data by at least one non-linear processing method, and wherein generating the quantification data comprises processing the second portion of the radio frequency data by at least one linear processing method.

8. The method (700) of claim 6, further comprising:
receiving a user input via the user interface (124) comprising a change to the image (406, 500, 600); and
adjusting the generating of the visualization data responsive to the user input.

9. The method (700) of claim 6, further comprising generating a second image (408, 502, 602) based on the quantification data.

10. The method (700) of claim 6, wherein the first imaging mode is a harmonic imaging mode, and the second imaging mode is a fundamental imaging mode.

## Patentansprüche

1. Ultraschallbildgebungssystem (100), das Folgendes umfasst:
eine Ultraschallsonde (112), die dafür ausgelegt ist, Ultraschallsignale zu senden und Echos in Reaktion auf die Ultraschallsignale zu empfangen und Hochfrequenzdaten (400) bereitzustellen, die den Echos entsprechen; und
einen Prozessor (126, 170, 172, 200), der dafür ausgelegt ist, die Hochfrequenzdaten zu empfangen, und ferner dafür ausgelegt ist: Visualisierungsdaten aus einem ersten Teil der Hochfrequenzdaten zu erzeugen, wobei ein Bild (406, 408, 500, 502, 600, 602) wenigstens teilweise auf den Visualisierungsdaten basiert; Quantifizierungsdaten aus einem zweiten Teil der Hochfrequenzdaten zu erzeugen; wobei die Quantifizierungsdaten medizinische Bildgebungsdaten für die Quantifizierung umfassen;
eine Anzeige (138), die dafür ausgelegt ist, das Bild anzuzeigen; und
eine Anwenderschnittstelle (124), die dafür ausgelegt ist, Angaben eines ersten interessierenden Bereichs (508) in dem Bild zu empfangen, wobei der erste interessierende Bereich einem Teil einer Leber (504) entspricht; Angaben eines zweiten interessierenden Bereichs (510) in dem Bild zu empfangen, wobei der zweite interessierende Bereich einem Teil einer Niere (506) entspricht;
und wobei der Prozessor ferner dafür ausgelegt ist: die Angaben des ersten und des zweiten interessierenden Bereichs zu empfangen; und einen physiologischen Parameter zu berechnen, wobei der physiologische Parameter einen hepatorenalen Index enthält;
und wobei das Berechnen des physiologischen Parameters das Berechnen eines Verhältnisses einer Echointensität der Leber und einer Echointensität der Niere wenigstens teilweise basierend auf einem Teil der Quantifizierungsdaten, die dem ersten interessierenden Bereich zugeordnet sind, und einem Teil der Quantifizierungsdaten, die dem zweiten interessierenden Bereich zugeordnet sind, umfasst;
**dadurch gekennzeichnet, dass** der erste Teil der Hochfrequenzdaten durch eine erste Bildgebungsbetriebsart erfasst wird und der zweite Teil der Hochfrequenzdaten durch eine zweite Bildgebungsbetriebsart erfasst wird, die sich von der ersten Bildgebungsbetriebsart unterscheidet; wobei die erste Bildgebungsbetriebsart mit der zweiten Bildgebungsbetriebsart verschachtelt ist.

2. Ultraschallbildgebungssystem (100) nach Anspruch 1,
wobei die Anwenderschnittstelle (124) ferner dafür ausgelegt ist, Eingaben zum Einstellen des Bildes (406, 408, 500, 502, 600, 602) zu empfangen, und
wobei der Prozessor (126, 200) ferner dafür ausgelegt ist, die Visualisierungsdaten basierend auf den Eingaben einzustellen.

3. Ultraschallbildgebungssystem (100) nach Anspruch 1, wobei die Anzeige (138) ferner dafür ausgelegt ist, ein zweites Bild anzuzeigen, das aus den Quantifizierungsdaten erzeugt wird.

4. Ultraschallsystem (100) nach Anspruch 1, wobei der Prozessor (126, 200) ferner dafür ausgelegt ist,
die Visualisierungsdaten durch das Verarbeiten des ersten Teils der Hochfrequenzdaten durch wenigstens ein nichtlineares Verarbeitungsverfahren zu erzeugen und
die Quantifizierungsdaten durch das Verarbeiten des zweiten Teils der Hochfrequenzdaten durch wenigstens ein lineares Verarbeitungsverfahren zu erzeugen.

5. Ultraschallbildgebungssystem (100) nach Anspruch 1, wobei die erste Bildgebungsbetriebsart eine Bildgebungsbetriebsart mit harmonischen Schwingungen ist und die zweite Bildgebungsbetriebsart eine Bildgebungsbetriebsart mit Grundschwingungen ist.

6. Ultraschallbildgebungsverfahren (700), das Folgendes umfasst:
Empfangen (702) von Hochfrequenzdaten (400), die Echos entsprechen, die in Reaktion auf Ultraschallsignale erzeugt werden, die durch eine Ultraschallumsetzeranordnung (114) gesendet werden;
Erzeugen (704) von Visualisierungsdaten aus einem ersten Teil (402) der Hochfrequenzdaten (400) durch einen Prozessor;
Erzeugen (706) von Quantifizierungsdaten aus einem zweiten Teil (404) der Hochfrequenzdaten (400) durch einen Prozessor,
wobei die Quantifizierungsdaten medizinische Bildgebungsdaten für die Quantifizierung umfassen;
Erzeugen (708) eines Bildes (406, 500, 600) durch eine Anzeige wenigstens teilweise basierend auf den Visualisierungsdaten;
Empfangen (710) einer Angabe eines ersten interessierenden Bereichs (508) in dem Bild von der Anwenderschnittstelle (124), wobei der erste interessierende Bereich einem Teil einer Leber entspricht (504);
Empfangen (710) einer Angabe eines zweiten interessierenden Bereichs (510) in dem Bild von einer Anwenderschnittstelle (124), wobei der zweite interessierende Bereich einem Teil einer Niere entspricht (506); und
Berechnen eines physiologischen Parameters, wobei der physiologische Parameter einen hepatorenalen Index umfasst, wobei das Berechnen des physiologischen Parameters das Berechnen eines Verhältnisses einer Echointensität der Leber und einer Echointensität der Niere wenigstens teilweise basierend auf einem Teil der Quantifizierungsdaten, die dem ersten interessierenden Bereich zugeordnet sind, und einem Teil der Quantifizierungsdaten, die dem zweiten interessierenden Bereich zugeordnet sind, umfasst,
**dadurch gekennzeichnet, dass**
der erste Teil der Hochfrequenzdaten durch eine erste Bildgebungsbetriebsart erfasst wird, und
der zweite Teil der Hochfrequenzdaten durch eine zweite Bildgebungsbetriebsart erfasst wird, die sich von der ersten Bildgebungsbetriebsart unterscheidet, wobei die erste Bildgebungsbetriebsart mit der zweiten Bildgebungsbetriebsart verschachtelt ist.

7. Verfahren (700) nach Anspruch 6, wobei das Erzeugen der Visualisierungsdaten das Verarbeiten des ersten Teils der Hochfrequenzdaten durch wenigstens ein nichtlineares Verarbeitungsverfahren umfasst und wobei das Erzeugen der Quantifizierungsdaten das Verarbeiten des zweiten Teils der Hochfrequenzdaten durch wenigstens ein lineares Verarbeitungsverfahren umfasst.

8. Verfahren (700) nach Anspruch 6, das ferner Folgendes umfasst:
Empfangen einer Anwendereingabe, die eine Änderung an dem Bild (406, 500, 600) umfasst, über die Anwenderschnittstelle (124); und
Einstellen des Erzeugens der Visualisierungsdaten in Reaktion auf die Anwendereingabe.

9. Verfahren (700) nach Anspruch 6, das ferner das Erzeugen eines zweiten Bildes (408, 502, 602) basierend auf den Quantifizierungsdaten umfasst.

10. Verfahren (700) nach Anspruch 6, wobei die erste Bildgebungsbetriebsart eine Bildgebungsbetriebsart mit harmonischen Schwingungen ist und die zweite Bildgebungsbetriebsart eine Bildgebungsbetriebsart mit Grundschwingungen ist.

## Revendications

1. Système d'imagerie ultrasonore (100) comprenant :
une sonde ultrasonore (112) configurée pour transmettre des signaux ultrasonores et recevoir des échos en réponse aux signaux ultrasonores et fournir des données radiofréquence (400) correspondant aux échos ; et
un processeur (126, 170, 172, 200) configuré pour recevoir les données radiofréquence et configuré en outre pour : générer des données de visualisation à partir d'une première partie des données radiofréquence, une image (406, 408, 500, 502, 600, 602) étant basée, au moins en partie, sur les données de visualisation ;
générer des données de quantification à partir d'une seconde partie des données radiofréquence ; les données de quantification comprenant des données d'imagerie médicale pour quantification ;
un dispositif d'affichage (138) configuré pour afficher l'image ; et
une interface utilisateur (124) configurée pour recevoir des indications d'une première région d'intérêt (508) dans l'image, la première région d'intérêt correspondant à une partie d'un foie (504) ; recevoir des indications d'une seconde région d'intérêt (510) dans l'image, la seconde région d'intérêt correspondant à une partie d'un rein (506) ;
et le processeur étant en outre configuré pour : recevoir les indications des première et seconde régions d'intérêt ; et calculer un paramètre physiologique, le paramètre physiologique comprenant un indice hépatorénal ;
et le calcul du paramètre physiologique comprenant le calcul d'un rapport d'une intensité d'écho du foie et d'une intensité d'écho du rein sur la base au moins en partie d'une partie des données de quantification associées à la première région d'intérêt et d'une partie des données de quantification associées à la seconde région d'intérêt ;
**caractérisé en ce que** la première partie des données radiofréquence est acquise par un premier mode d'imagerie et la seconde partie des données radiofréquence est acquise par un second mode d'imagerie différent du premier mode d'imagerie, le premier mode d'imagerie étant entrelacé avec le second mode d'imagerie.

2. Système d'imagerie ultrasonore (100) selon la revendication 1, l'interface utilisateur (124) étant en outre configurée pour recevoir des entrées pour ajuster l'image (406, 408, 500, 502, 600, 602), et
le processeur (126, 200) étant en outre configuré pour ajuster les données de visualisation sur la base des entrées.

3. Système d'imagerie ultrasonore (100) selon la revendication 1, le dispositif d'affichage (138) étant en outre configuré pour afficher une seconde image générée à partir des données de quantification.

4. Système d'imagerie ultrasonore (100) selon la revendication 1, le processeur (126, 200) étant en outre configuré pour :
générer les données de visualisation en traitant la première partie des données radiofréquence par au moins un procédé de traitement non linéaire, et
générer les données de quantification en traitant la seconde partie des données radiofréquence par au moins un procédé de traitement linéaire.

5. Système d'imagerie ultrasonore (100) selon la revendication 1, le premier mode d'imagerie étant un mode d'imagerie harmonique, et le second mode d'imagerie étant un mode d'imagerie fondamental.

6. Procédé d'imagerie ultrasonore (700) comprenant :
la réception (702) de données radiofréquence (400) correspondant à des échos générés en réponse à des signaux ultrasonores émis par un réseau de transducteurs ultrasonores (114) ;
la génération (704) de données de visualisation à partir d'une première partie (402) des données radiofréquence (400) par un processeur ;
la génération (706) de données de quantification à partir d'une seconde partie (404) des données radiofréquence (400) par un processeur,
les données de quantification comprenant des données d'imagerie médicale pour quantification ;
la génération (708) d'une image (406, 500, 600) par un dispositif d'affichage à partir, au moins en partie, des données de visualisation ;
la réception (710) d'une indication d'une première région d'intérêt (508) dans l'image provenant de l'interface utilisateur (124), la première région d'intérêt correspondant à une partie d'un foie (504) ;
la réception (710) d'une indication d'une seconde région d'intérêt (510) dans l'image à partir d'une interface utilisateur (124), la seconde région d'intérêt correspondant à une partie d'un rein (506) ; et
le calcul d'un paramètre physiologique, le paramètre physiologique comprenant un indice hépatorénal, le calcul du paramètre physiologique comprenant le calcul d'un rapport d'une intensité d'écho du foie et d'une intensité d'écho du rein sur la base au moins en partie d'une partie des données de quantification associées à la première région d'intérêt et d'une partie des données de quantification associées à la seconde région d'intérêt, **caractérisée en ce que**
la première partie des données radiofréquence est acquise par un premier mode d'imagerie, et
la seconde partie des données radiofréquence est acquise par un second mode d'imagerie différent du premier mode d'imagerie, le premier mode d'imagerie étant entrelacé avec le second mode d'imagerie.

7. Procédé (700) selon la revendication 6, la génération des données de visualisation comprenant le traitement de la première partie des données radiofréquence par au moins un procédé de traitement non linéaire, et la génération des données de quantification comprenant le traitement de la seconde partie des données radiofréquence par au moins un procédé de traitement linéaire.

8. Procédé (700) selon la revendication 6, comprenant en outre l'étape suivante :
la réception d'une entrée utilisateur par l'intermédiaire de l'interface utilisateur (124) comprenant une modification de l'image (406, 500, 600) ; et
l'ajustement de la génération des données de visualisation en réponse à l'entrée utilisateur.

9. Procédé (700) selon la revendication 6, comprenant en outre la génération d'une seconde image (408, 502, 602) sur la base des données de quantification.

10. Procédé (700) selon la revendication 6, le premier mode d'imagerie étant un mode d'imagerie harmonique, et le second mode d'imagerie étant un mode d'imagerie fondamental.
